# FASCICULE DE BREVET EUROPEEN

(11) **EP 2 580 186 B1**
(45) Date de publication et mention de la délivrance du brevet: **16.08.2017**
(21) Numéro de dépôt: 11734161.0
(22) Date de dépôt: 09.06.2011
(51) Int. Cl.: C07C 231/02, C07C 231/14

(54) **PROCEDE DE PREPARATION DE COMPOSES ESTERAMIDES**
VERFAHREN ZUR HERSTELLUNG VON ESTERAMIDVERBINDUNGEN
PROCESS FOR PREPARING ESTERAMIDE COMPOUNDS

(30) Priorité: 09.06.2010 FR 1054534
(43) Date de publication de la demande: 17.04.2013
(73) Titulaire: Rhodia Operations, 93306 Aubervilliers (FR)
(72) Inventeur: VIDAL, Thierry, F-69003 Lyon (FR); RACHED, Rabih, F-69390 Millery (FR); GUGLIERI, Massimo, MC-98000 Monaco (MC)
(74) Mandataire: Cardon, Flavie
(86) Numéro de dépôt international: PCT/FR2011/051315
(87) Numéro de publication internationale: WO 2011/154661

(56) Documents cités:
- WO-A1-2009/092795

## Description

La présente invention concerne un procédé de préparation de composés esteramides.

Plus particulièrement, l'invention est relative à un procédé de préparation de composés esteramides par réaction entre un diester et une amine.

Les composés esteramides sont connus pour leurs applications comme solvants notamment dans des applications phytosanitaires, comme décrit, par exemple, dans le document WO 2009/092795.

Il existe plusieurs voies d'accès aux dits composés esteramides.

Le document US 4588833 décrit un procédé de préparation d'esteramides par réaction à haute température, catalysée par le cobalt, d'un amide insaturé avec un alcool et du monoxyde de carbone.

Le document US 3417114 décrit dans l'exemple 9, un procédé de préparation simultanée d'un composé esteramide « DMGME » de formule : MeOOC-(CH₂)₃-CONMe₂ et d'un composé diamide « TMG » de formule : Me₂NOC-(CH₂)₃-CONMe₂, suivie de la séparation par distillation de ces deux composés. Pour faire la réaction, de la diméthylamine gazeuse est mise à buller pendant deux heures dans un milieu comprenant du glutarate de diméthyle préalablement purifié et une solution de méthylate de sodium. Les deux composés (DMGE et TMG) sont ensuite isolés par distillation à partir du mélange complexe obtenu.

Le document US 3288794 décrit le même procédé que le document US 3417114 ainsi que la préparation simultanée d'ester méthylique de N,N-diméthyl-adipamide et de N,N,N',N'-tétraméthyl-adipamide avec un mode opératoire similaire, suivie également d'une séparation par distillation.

Dans le cas des diesters, les procédés de l'art antérieur ci-dessus décrits, ne sont pas sélectifs en esteramide. La proportion de diamide est d'ailleurs souvent majoritaire et l'esteramide est considéré comme un sous-produit qui n'a pas totalement réagi.

En outre, pour de tels procédés, une étape de purification du diester préalablement à la réaction est nécessaire, ce qui complique le procédé.

Ce type de procédé nécessite également des traitements lourds et coûteux du milieu réactionnel après réaction pour isoler le diamide et l'esteramide, comme par exemple des distillations.

De plus, avec ces procédés de l'art antérieur, les produits bruts formés peuvent présenter une forte coloration jaune-orangée qui est gênante pour les utilisations ultérieures. Les produits sont donc soumis à des étapes supplémentaires de purification. Tous ces traitements de purification complexifient les procédés de fabrication des esteramides.

Pour tenter de palier le problème de la faible sélectivité en esteramide, il est courant de travailler à des températures basses, c'est-à-dire inférieures à la température ambiante, en refroidissant le milieu réactionnel. Cependant, l'abaissement de la température de réaction diminue significativement la cinétique de la réaction et donc la productivité du procédé.

Il existe donc un besoin de trouver un procédé de fabrication de composés esteramides à partir de diesters qui soit sélectif en esteramide. En outre, le procédé doit être facile à mettre en oeuvre dans une installation industrielle. Un autre impératif auquel doit répondre le procédé est que la cinétique de la réaction doit être grande. Enfin, le procédé de fabrication d'esteramide doit être productif.

A cet effet, la présente invention propose un procédé de préparation d'un composé esteramide de formule (I) suivante :

R¹OOC-A-CONR²R³ (I)

comprenant une étape de réaction entre :
un composé diester de formule (II) suivante :

   R¹OOC-A-COOR¹ (II)

   et une amine de formule (III) suivante :

   HNR²R³ (III)

   en présence d'un composé basique,
   formules dans lesquelles :
   - A est une liaison covalente ou un groupe alkylène divalent linéaire ou ramifié comprenant un nombre d'atomes de carbone allant de 1 à 12,
   - R¹ est un groupe hydrocarboné éventuellement substitué, comprenant de 1 à 36 atomes de carbone,
   - R² et R³, identiques ou différents, sont des groupes choisis parmi l'atome d'hydrogène et les groupes hydrocarbonés, éventuellement substitués, comprenant de 1 à 36 atomes de carbone,
   - R² et R³ pouvant former ensemble un cycle comprenant l'atome d'azote auquel ils sont liés, ledit cycle étant le cas échéant substitué et/ou comprenant un hétéroatome supplémentaire, et
   - R² et R³ n'étant pas simultanément des atomes d'hydrogène,
   caractérisé par le fait que
   - l'amine (III) est solubilisée dans un solvant organique ou dans le composé diester (II),
   - lorsque l'amine (III) est solubilisée dans un solvant organique, le composé diester (II) est additionné sur le mélange réactionnel comprenant l'amine (III) et le composé basique,
   - lorsque l'amine (III) est solubilisée dans le composé diester (II), le composé basique est additionné sur le mélange réactionnel comprenant l'amine (III) et le composé diester (II),
   - la réaction est conduite à une température comprise entre 30°C et 130°C,
   - l'amine (III) est présente en un excès molaire allant de 0,01 à 50 %, par rapport au composé diester (II).

Selon le procédé de l'invention, l'amine (III) est toujours présente dans le milieu réactionnel sur lequel l'un des composés (diester ou basique) est additionné, l'autre composé (basique ou diester) étant présent avec l'amine dès le début de la réaction.

Intervient donc dans le procédé de l'invention un composé diester de formule (II), qui présente avantageusement les caractéristiques ci-dessous.

Selon un mode avantageux de l'invention, dans les formules (I) et (II), A est un groupe alkylène divalent ramifié comprenant un nombre d'atomes de carbone allant de 2 à 12, de préférence allant de 3 à 6 atomes de carbone.

De façon préférée, dans les formules (I) et (II), les groupes R¹, identiques ou différents, sont des groupes hydrocarbonés comprenant de 1 à 16 atomes de carbone et pouvant porter un ou plusieurs substituants. Par « substituant » on entend, à titre illustratif et sans caractère limitatif, un groupe alkyle ayant de préférence de 1 à 4 atomes de carbone, alcoxy ayant de préférence de 1 à 4 atomes de carbone, hydroxy ou halogéno.

Préférentiellement, les groupes R¹, identiques ou différents, sont choisis parmi les groupes alkyle, alcényle, cycloalkyle, aryle et arylalkyle, lesdits groupes pouvant porter un ou plusieurs substituants.

Plus particulièrement R¹ est préférentiellement choisi parmi les groupes méthyle, éthyle, n-propyle, isopropyle, n-butyle, isobutyle, tertiobutyle, sec-butyle, n-pentyle, isopentyle, sec-pentyle, cyclopentyle, n-hexyle, isohexyle, sec-hexyle, cyclohexyle, méthylcyclohexyle, 2-éthylbutyle, 3-méthylpentyle, n-heptyle, isoheptyle, sec-heptyle, 3-méthylhexyle, 4-méthylhexyle, 1-éthylpentyle, 2-éthylpentyle, 3-éthylpentyle, n-octyle, isooctyle, 3-méthylheptyle, n-nonyle, n-décyle, undécyle, n-dodécyle, tridécyle, tétradécyle et pentadécyle.

Dans un mode particulièrement avantageux, R¹ est choisi parmi les groupes méthyle et éthyle.

Tout préférentiellement, le composé diester de formule (II) est un mélange de composés diesters de formules (II.1), (II.2) et (II.3) suivantes :

R¹OOC-CH(CH₃)-CH₂-CH₂-COOR¹ (II.1)

R¹OOC-CH(CH₂-CH₃)-CH₂-COOR¹ (II.2)

R¹OOC-CH₂-CH₂-CH₂-CH₂-COOR¹ (II.3)

avec R¹ tel que défini ci-dessus.

Le mélange de composés diesters de formule (11.1), (II.2) et (II.3) peut présenter la composition suivante :
- de 75 à 95 % en poids de composé de formule (11.1), de préférence de 85 à 95 % en poids,
- de 3 à 23 % en poids de composé de formule (II.2), de préférence de 4 à 14 % en poids,
- de 0,1 à 10 % en poids de composé de formule (II.3), de préférence de 0,1 à 3 % en poids.

Il est particulièrement préféré que pour le mélange de composés diesters de formule (II.1), (II.2) et (II.3) ci-dessus les groupes R¹ soient des groupes méthyle. Il peut s'agir notamment d'un mélange de diesters commercialisé par Rhodia sous la dénomination Rhodiasolv® IRIS.

Selon un mode avantageux de l'invention, le composé diester (II) est introduit pur, c'est-à-dire qu'il n'est pas mis en solution dans un solvant organique. Toutefois, il est possible que le composé diester (II) soit mis en solution dans un solvant organique. Selon un mode préféré de réalisation de l'invention, le solvant organique est choisi parmi les solvants organiques volatils, notamment les alcools et les éthers, de préférence parmi le méthanol, l'éthanol, le tétrahydrofurane (THF) et leurs mélanges. Plus préférentiellement, le solvant organique est le méthanol.

Intervient également dans le procédé de l'invention une amine de formule (III), qui présente avantageusement les caractéristiques ci-dessous.

De façon préférée, dans les formules (I) et (III), les groupes R² et R³, identiques ou différents, sont des groupes hydrocarbonés comprenant de 1 à 16 atomes de carbone pouvant porter un ou plusieurs substituants. Par « substituant » on entend, à titre illustratif et sans caractère limitatif, un groupe alkyle ayant de préférence de 1 à 4 atomes de carbone, alcoxy ayant de préférence de 1 à 4 atomes de carbone, hydroxy ou halogéno.

Préférentiellement, les groupes R² et R³, identiques ou différents, sont choisis parmi les groupes alkyle, alcényle, cycloalkyle, aryle et arylalkyle, lesdits groupes pouvant porter un ou plusieurs substituants.

Selon un premier mode de réalisation de l'invention, R² et R³, identiques ou différents, sont choisis parmi les groupes méthyle, éthyle, hydroxyéthyle, n-propyle, isopropyle, n-butyle, isobutyle, tertiobutyle, n-pentyle, isoamyle, hexyle et cyclohexyle. De préférence, R² et R³ sont choisis parmi les groupes méthyle, éthyle et hydroxyéthyle.

Selon un second mode de réalisation de l'invention, R² et R³ forment ensemble un cycle à 5 ou 6 atomes comprenant l'atome d'azote auquel ils sont liés, l'un des atomes du cycle pouvant être un autre hétéroatome, tel que par exemple l'oxygène. De préférence, R² et R³ forment ensemble un cycle étant choisi parmi une morpholine, une pipéridine et une pipérazine.

L'amine de formule (III) est solubilisée dans un solvant organique ou dans le composé diester (II).

Selon un mode préféré de réalisation de l'invention, le solvant organique est choisi parmi les solvants organiques volatils, notamment les alcools et les éthers, de préférence parmi le méthanol, l'éthanol, le tétrahydrofurane (THF) et leurs mélanges. Plus préférentiellement, le solvant organique est le méthanol.

L'amine de formule (III) est présente à une concentration en poids dans le solvant organique qui est avantageusement comprise entre 10 et 100 %, de préférence entre 30 et 80 % et encore plus préférentiellement entre 40 et 60 %. Le cas où l'amine (III) est présente dans une concentration de 100 % correspond au mode dans lequel l'amine est solubilisée dans le composé diester (II), et qu'il n'y a pas de solvant organique additionnel.

Le procédé de l'invention met en jeu un composé basique.

De préférence, le composé basique est un alcoxyde de métal alcalin ou alcalino-terreux, de préférence choisi parmi le méthylate de sodium, l'éthylate de sodium ou de potassium, le ter-butylate de potassium. Le composé basique peut également être choisi parmi les carbonates, notamment le carbonate de potassium ou de sodium ; ou les titanates d'alkyle, comme par exemple le titanate de butyle. Le composé basique peut également être un mélange de plusieurs composés ci-dessus cités. De préférence, le composé basique est le méthylate de sodium.

Selon l'invention, le composé basique peut être utilisé pur ou en solution dans un solvant organique, par exemple de nature identique à celui précédemment défini, notamment le méthanol. Généralement, le composé basique est en solution dans un solvant organique à une concentration en poids dans le solvant organique qui est avantageusement comprise entre 5 et 80 %, de préférence entre 10 et 50 % et encore plus préférentiellement entre 20 et 30 %.

Conformément à l'invention, on fait réagir le composé diester de formule (II) et l'amine de formule (III) en présence du composé basique, dans les proportions définies ci-après.

Selon une caractéristique de l'invention, l'amine (III) est présente dans un excès molaire allant de 0,01 à 50 %, par rapport au composé diester (II). De préférence, l'amine est présente en un excès molaire allant de 1 à 30 % et encore plus préférentiellement de 5 à 20 %, par rapport au composé diester (II). La stoechiométrie pour obtenir le composé esteramide de formule (I) étant de 1 mole d'amine de formule (III) par mole de composé diester de formule (II), on entend ici par « excès molaire » que le procédé de l'invention fait intervenir un excès molaire d'amine allant de 0,01 à 50 % par rapport à cette quantité stoechiométrique.

Le composé basique est de préférence introduit à une concentration molaire par rapport au diester comprise entre 0,01 et 20 %, de préférence entre 3 et 10 %.

Selon une autre caractéristique de l'invention, la réaction est conduite à une température comprise entre 30°C et 130°C. De préférence, la réaction est conduite à une température supérieure ou égale à 50°C. De façon avantageuse, la réaction est conduite à une température comprise entre 40°C et 90°C et encore plus préférentiellement entre 45°C et 65°C. Les calories nécessaires au contrôle de cette température sont apportées soit par la réaction elle-même, soit par un moyen externe, par exemple de chauffage ou de refroidissement.

De façon avantageuse, la réaction est conduite à une pression comprise entre 1 et 10 bars absolus, de préférence entre 1 et 5 bars absolus et encore plus préférentiellement entre 1 et 3 bars absolus.

De façon préférée, la réaction est conduite dans des conditions anhydres, c'est-à-dire que l'on tolère jusqu'à 0,2 % en poids d'eau, de préférence jusqu'à 0,05 % en poids d'eau.

Selon un mode préféré, la réaction est conduite dans des conditions inertes, par exemple au moyen d'un balayage avec un gaz inerte, notamment de l'azote.

Selon l'invention, le composé (diester ou basique) qui est additionné sur le mélange réactionnel comprenant l'amine (III), peut être additionné en une seule fois, en continu ou par portions.

Conformément à l'invention, on obtient en fin de réaction le composé esteramide de formule (I). Le mélange réactionnel en fin de réaction ne nécessite pas de traitement lourd de purification du composé esteramide pour le séparer du composé diamide. Seuls les composés volatils comme l'amine n'ayant pas réagi et le solvant peuvent être éliminés, notamment évaporés par exemple par distillation sous pression réduite. Le milieu peut éventuellement être traité par des opérations classiques connues de l'homme du métier, notamment des étapes de neutralisation, filtration, et lavage pour éliminer les sels formés en cours de réaction. Le composé esteramide de formule (I) ainsi obtenu présente une grande pureté et peut être directement utilisé dans les applications auxquelles il est destiné, par exemple comme solvant, notamment dans des applications phytosanitaires.

Le procédé selon l'invention peut être un procédé continu ou discontinu.

Le procédé selon l'invention présente de nombreux avantages. Il est tout particulièrement avantageux lorsqu'il est mis en oeuvre sur un mélange de composés diesters répondant aux formules (11.1), (II.2) et (II.3), par exemple Rhodiasolv®IRIS, en présence d'un excès molaire d'amine (III) par rapport au mélange de diester et à une température supérieure à 30°C.

Tout d'abord, il est très sélectif en esteramide, c'est-à-dire que la sélectivité en esteramide est supérieure à 90 %, voire supérieure à 95 %. De façon surprenante, il se forme moins de 5 % de diamide malgré l'excès d'amine et une température de réaction supérieure ou égale à 30°C. La réaction est également très rapide comparée à des réactions à plus faible température et sans excès d'amine. En outre, aucune coloration significative du milieu n'est observée et le mélange réactionnel en fin de réaction ne nécessite pas de traitement lourd de purification du produit majoritaire, ce qui simplifie grandement le procédé et augmente sa productivité.

Les exemples qui suivent, illustrent l'invention sans toutefois la limiter.

### Exemples

### Exemple 1 (comparatif) : Diméthylamine (gaz) sur mélange Rhodiasolv® IRIS / méthylate de sodium - T = 20°C

Dans un réacteur double enveloppé parfaitement agité de 1 L, muni d'une agitation mécanique et préalablement séché, sont placés une solution de méthylate de sodium dans le méthanol (solution à 50 % poids : poids, 18,8 g) et 304 g d'un mélange de diesters d'acide 2-méthyl-glutarique, d'acide 2-éthyl-succinique et d'acide adipique commercialisé sous la dénomination Rhodiasolv® IRIS par Rhodia.
On constate que le milieu réactionnel se colore en jaune orangé.
La température du milieu réactionnel est stabilisée à la consigne de +20°C et de la diméthylamine gazeuse est mise à buller pendant 2 heures dans le mélange réactionnel comprenant le méthylate de sodium et le mélange de diesters (addition de 82 g de diméthylamine, excès molaire de 5% par rapport au diester).
La température du milieu réactionnel est maintenue constante pendant la durée du bullage.
La température du milieu réactionnel est maintenue à +20°C jusqu'à ce que 96 % du diester initialement introduit ait été consommé (durée de finition : 20 heures).
Le léger excès de diméthylamine est distillé sous pression réduite (∼200 mbar) à une température comprise entre 20 et 50°C. Le milieu réactionnel est alors neutralisé par ajout de la quantité suffisante d'acide phosphorique à 85 %. Les sels formés sont filtrés. Le solide formé est lavé par du méthanol puis les composés volatils (méthanol et diester non réagi) sont distillés sous vide.
Le produit est alors analysé par chromatographie en phase gazeuse.

Le tableau 1 ci-après rassemble les conditions et résultats de cet exemple 1C comparatif.

**Tableau 1**

| Essai | T (°C) | Durée (h)^{(a)} | Esteramide (%)^{(b)} | Diamide (%)^{(b)} | Di/EA ^{(c)} | Coloration du milieu réactionnel brut |
|---|---|---|---|---|---|---|
| 1C | 20 | 20 | 95 | 3 | 3,1 | Jaune-orangé |

| | | | | | | |
|---|---|---|---|---|---|---|
| (a) Durée nécessaire à l'achèvement de la réaction (b) Composition en espèce citée à la fin du traitement (c) Di/EA = rapport des concentrations finales diamide/esteramide | | | | | | |

On constate que la réaction est lente et que le produit obtenu est coloré.

### Exemple 2 - Méthylate de sodium sur mélange Rhodiasolv® IRIS / Diméthylamine - Influence de la température

Dans un réacteur double enveloppé parfaitement agité de 1 L, muni d'une agitation mécanique et préalablement séché, est introduite de la diméthylamine (DMA pure, 83 g, excès molaire de 5 % par rapport au diester) solubilisée dans 304 g d'un mélange de diesters d'acide 2-méthyl-glutarique, d'acide 2-éthyl-succinique et d'acide adipique commercialisé sous la dénomination Rhodiasolv® IRIS par Rhodia.
La température du milieu réactionnel est stabilisée à la consigne désirée (cf. tableau 2 ci-dessous) et une solution de méthylate de sodium dans le méthanol (solution à 50 % poids: poids, 18,8 g) est coulée sur le mélange réactionnel comprenant la diméthylamine et le mélange de diesters en une durée définie.
Une fois la coulée achevée, la température du milieu réactionnel est maintenue constante jusqu'à consommation de plus de 96 % de la charge en diester engagée.
L'excès de diméthylamine est distillé sous pression réduite (∼200 mbar) à une température comprise entre 20 et 50°C. Le milieu réactionnel est alors neutralisé par ajout de la quantité suffisante d'acide phosphorique à 85 %. Les sels formés sont filtrés. Le solide résiduel est lavé par du méthanol puis les composés volatils sont distillés sous vide.
Le produit est alors analysé par chromatographie en phase gazeuse.

Le tableau 2 ci-dessous rassemble les conditions et résultats des deux essais mis en oeuvre (essai 2 C : comparatif et essai 2 : selon l'invention).

**Tableau 2**

| Essai | DMA (% mol) | T (°C) | Durée (h) ^{(a)} | Esteramide (%)^{(b)} | Diamide (%)^{(b)} | Di/EA (%)^{(c)} |
|---|---|---|---|---|---|---|
| 2C | 100 | 20 | 8 | 95,8 | 4 | 4,2 |
| 2 | 100 | 50 | 1 | 96 | 4,2 | 4,4 |

| | | | | | | |
|---|---|---|---|---|---|---|
| (a) Durée nécessaire à l'achèvement de la réaction (b) Composition en espèce citée à la fin du traitement (c) Di/EA = rapport des concentrations finales diamide/esteramide | | | | | | |

On constate que la réaction est lente pour l'exemple comparatif 2C alors qu'elle est très rapide pour l'exemple 2 selon l'invention dans lequel la sélectivité est conservée malgré l'augmentation de la température à une valeur de 50 °C.

### Exemple 3 - Rhodiasolv® IRIS sur mélange méthylate de sodium / Diméthylamine - Influence de la température - Influence de l'excès de DMA

Dans un réacteur double enveloppé parfaitement agité de 1 L, muni d'une agitation mécanique et préalablement séché, sont placés la diméthylamine dans le méthanol (solution à 50 % poids : poids, 188 g) et le méthylate de sodium dans le méthanol (solution à 50 % poids : poids, 18,8 g).
La température du milieu réactionnel est stabilisée à la consigne désirée et 304 g d'un mélange de diesters d'acide 2-méthyl-glutarique, d'acide 2-éthyl-succinique et d'acide adipique commercialisé sous la dénomination Rhodiasolv® IRIS par Rhodia est coulé sur le mélange réactionnel comprenant la diméthylamine et le méthylate de sodium en une durée définie.
Une fois la coulée achevée, la température du milieu réactionnel est maintenue constante jusqu'à consommation de plus de 96 % de la charge en diester engagée.
L'excès de diméthylamine est distillé sous pression réduite (∼200 mbar) à une température comprise entre 20 et 50°C. Le milieu réactionnel est alors neutralisé par ajout de la quantité suffisante d'acide phosphorique à 85 %. Les sels formés sont filtrés. Le solide résiduel est lavé par du méthanol puis les composés volatils sont distillés sous vide.
Le produit est alors analysé par chromatographie en phase gazeuse.

Les tableaux 3 et 4 ci-dessous rassemblent les conditions et résultats des différents essais mis en oeuvre (C correspond à un essai comparatif).

### Effet de la Température

Diester/ DMA/ MeONa = 1 mol / 1,2 mol / 0,05 mol - DMA à 50 % poids dans le méthanol

**Tableau 3**

| Essai | T (°C) | Durée (h) ^{(a)} | Esteramide (%)^{(b)} | Diamide (%)^{(b)} | Di/EA (%)^{(c)} |
|---|---|---|---|---|---|
| 3.1C | 15 | 20 | 96 | 4 | 4,2 |
| 3.2C | 20 | 12 | 95,8 | 4,2 | 4,4 |
| 3.3 | 40 | 6 | 96,2 | 3,8 | 3,9 |
| 3.4 | 50 | 3 | 95,5 | 4,5 | 4,7 |

| | | | | | |
|---|---|---|---|---|---|
| (a) Durée nécessaire à l'achèvement de la réaction (b) Composition en espèce citée à la fin du traitement (c) Di/EA = rapport des concentrations finales diamide/esteramide | | | | | |

On constate que la vitesse de réaction est considérablement réduite dès 40 °C, tout en conservant la sélectivité en esteramide.

### Effet de la stoechiométrie en DMA

Diester/ MeONa = 1 mol / 0,05 mol - DMA à 50 % poids dans le méthanol

**Tableau 4**

| Essai | DMA (%)^{(c)} | T (°C) | Durée (h)^{(a)} | Esteramide (%)^{(b)} | Diamide (%)^{(b)} | Di/EA (%)^{(d)} | Commentaire |
|---|---|---|---|---|---|---|---|
| 3.5C | 0 | 20 | > 24 | 96,9 | 3 | 3 | Conversion < 96% après 24 heures |
| 3.6 | 0 | 50 | > 24 | 96 | 3,7 | 3,8 | Conversion < 96% après 24 heures |
| 3.7C | 10 | 20 | > 24 | 96,5 | 3 | 3,1 | Conversion < 96% après 24 heures |
| 3.8 | 10 | 50 | 7,5 | 96 | 4,8 | 5 | |
| 3.2C | 20 | 20 | 12 | 95,8 | 4,2 | 4,4 | |
| 3.4 | 20 | 50 | 3 | 95,5 | 4,5 | 4,7 | |

| | | | | | | | |
|---|---|---|---|---|---|---|---|
| (a) Durée nécessaire à l'achèvement de la réaction (b) Composition en espèce citée à la fin du traitement (c) Excès molaire de DMA par rapport au diester (% molaire) (d) Di/EA = rapport des concentrations finales en diamide/esteramide | | | | | | | |

On constate que sans excès de DMA, la réaction est très lente. L'excès de DMA, de façon surprenante, permet de conserver la sélectivité en esteramide à une température de 50°C. Une telle température de 50°C favorise en outre la cinétique de la réaction.

## Revendications

1. Procédé de préparation d'un composé esteramide de formule (I) suivante :
R¹OOC-A-CONR²R³ (I)
comprenant une étape de réaction entre :
un composé diester de formule (II) suivante :
R¹OOC-A-COOR¹ (II)
et une amine de formule (III) suivante :
HNR²R³ (III)
en présence d'un composé basique,
formules dans lesquelles :
- A est une liaison covalente ou un groupe alkylène divalent linéaire ou ramifié comprenant un nombre d'atomes de carbone allant de 1 à 12,
- R¹ est un groupe hydrocarboné éventuellement substitué, comprenant de 1 à 36 atomes de carbone,
- R² et R³, identiques ou différents, sont des groupes choisis parmi l'atome d'hydrogène et les groupes hydrocarbonés, éventuellement substitués, comprenant de 1 à 36 atomes de carbone,
- R² et R³ pouvant former ensemble un cycle comprenant l'atome d'azote auquel ils sont liés, ledit cycle étant le cas échéant substitué et/ou comprenant un hétéroatome supplémentaire, et
- R² et R³ n'étant pas simultanément des atomes d'hydrogène,
**caractérisé par le fait que**
- l'amine (III) est solubilisée dans un solvant organique ou dans le composé diester (II),
- lorsque l'amine (III) est solubilisée dans un solvant organique, le composé diester (II) est additionné sur le mélange réactionnel comprenant l'amine (III) et le composé basique,
- lorsque l'amine (III) est solubilisée dans le composé diester (II), le composé basique est additionné sur le mélange réactionnel comprenant l'amine (III) et le composé diester (II),
- la réaction est conduite à une température comprise entre 30°C et 130°C,
- l'amine (III) est présente en un excès molaire allant de 0,01 à 50 %, par rapport au composé diester (II).

2. Procédé selon la revendication 1, dans lequel A est un groupe alkylène divalent ramifié comprenant un nombre d'atomes de carbone allant de 2 à 12, de préférence allant de 3 à 6 atomes de carbone.

3. Procédé selon la revendication 1 ou 2, dans lequel les groupes R¹, identiques ou différents, sont des groupes hydrocarbonés comprenant de 1 à 16 atomes de carbone pouvant porter un ou plusieurs substituants.

4. Procédé selon l'une des revendications 1 à 3, dans lequel les groupes R¹, identiques ou différents, sont choisis parmi les groupes alkyle, alcényle, cycloalkyle, aryle et arylalkyle, lesdits groupes pouvant porter un ou plusieurs substituants.

5. Procédé selon l'une quelconque des revendications 1 à 4, dans lequel R¹ est choisi parmi les groupes méthyle, éthyle, n-propyle, isopropyle, n-butyle, isobutyle, tertiobutyle, sec-butyle, n-pentyle, isopentyle, sec-pentyle, cyclopentyle, n-hexyle, isohexyle, sec-hexyle, cyclohexyle, méthylcyclohexyle, 2-éthylbutyle, 3-méthylpentyle, n-heptyle, isoheptyle, sec-heptyle, 3-méthylhexyle, 4-méthylhexyle, 1-éthylpentyle, 2-éthylpentyle, 3-éthylpentyle, n-octyle, isooctyle, 3-méthylheptyle, n-nonyle, n-décyle, undécyle, n-dodécyle, tridécyle, tétradécyle et pentadécyle.

6. Procédé selon l'une quelconque des revendications 1 à 5, dans lequel le composé diester de formule (II) est un mélange de composés diesters de formules (11.1), (II.2) et (II.3) suivantes :
R¹OOC-CH(CH₃)-CH₂-CH₂-COOR¹ (II.1)
R¹OOC-CH(CH₂-CH₃)-CH₂-COOR¹ (II.2)
R¹OOC-CH₂-CH₂-CH₂-CH₂-COOR¹ (II.3)
avec R¹ tel que défini à l'une quelconque des revendications 1 à 5.

7. Procédé selon la revendication 6, dans lequel le mélange de composés diesters de formule (II.1), (II.2) et (II.3) présente la composition suivante :
- de 75 à 95 % en poids de composé de formule (11.1), de préférence de 85 à 95 % en poids,
- de 3 à 23 % en poids de composé de formule (II.2), de préférence de 4 à 14 % en poids,
- de 0,1 à 10 % en poids de composé de formule (II.3), de préférence de 0,1 à 3 % en poids.

8. Procédé selon la revendication 6 ou 7, dans lequel les groupes R¹ sont des groupes méthyle.

9. Procédé selon la revendication 1, dans lequel les groupes R² et R³, identiques ou différents, sont choisis parmi les groupes alkyle, alcényle, cycloalkyle, aryle et arylalkyle, lesdits groupes pouvant porter un ou plusieurs substituants.

10. Procédé selon la revendication 9, dans lequel R² et R³, identiques ou différents, sont choisis parmi les groupes méthyle, éthyle, hydroxyéthyle, n-propyle, isopropyle, n-butyle, isobutyle, tertiobutyle, n-pentyle, isoamyle, hexyle et cyclohexyle.

11. Procédé selon la revendication 1, dans lequel R² et R³ forment ensemble un cycle à 5 ou 6 atomes comprenant l'atome d'azote auquel ils sont liés, l'un des atomes du cycle pouvant être un autre hétéroatome, tel que par exemple l'oxygène.

12. Procédé selon la revendication 11, dans lequel R² et R³ forment ensemble un cycle choisi parmi une morpholine, une pipéridine et une pipérazine.

13. Procédé selon l'une des revendications 1 à 12, dans lequel l'amine de formule (III) est solubilisée dans un solvant organique.

14. Procédé selon la revendication 13, dans lequel le solvant organique est choisi parmi les alcools et les éthers, de préférence parmi le méthanol, l'éthanol, le tétrahydrofurane (THF) et leurs mélanges.

15. Procédé selon l'une des revendications 1 à 14, dans lequel le composé basique est un alcoxyde de métal alcalin ou alcalino-terreux, de préférence choisi parmi le méthylate de sodium, l'éthylate de sodium ou de potassium, le ter-butylate de potassium ; le carbonate de potassium ou de sodium, les titanates d'alkyle et leurs mélanges.

16. Procédé selon l'une des revendications 1 à 15, dans lequel le composé basique est introduit à une concentration molaire par rapport au diester comprise entre 0,01 et 20 %, de préférence entre 3 et 10 %.

17. Procédé selon l'une des revendications 1 à 16, dans lequel la réaction est conduite à une température comprise entre 40°C et 90°C et encore plus préférentiellement entre 45°C et 65°C.

## Patentansprüche

1. Verfahren zur Herstellung einer Esteramidverbindung der folgenden Formel (I) :
R¹OOC-A-CONR²R³ (I)
umfassend einen Schritt der Umsetzung
einer Diesterverbindung der folgenden Formel (II) :
R¹OOC-A-COOR¹ (II)
mit einem Amin der folgenden Formel (III) :
HNR²R³ (III)
in Gegenwart einer basischen Verbindung,
wobei in den Formeln :
- A für eine kovalente Bindung oder eine lineare oder verzweigte zweiwertige Alkylengruppe mit einer Zahl von Kohlenstoffatomen im Bereich von 1 bis 12 steht,
- R¹ für eine gegebenenfalls substituierte Kohlenwasserstoffgruppe mit 1 bis 36 Kohlenstoffatomen steht,
- R² und R³ gleich oder verschieden sind und für Gruppen, die aus dem Wasserstoffatom und gegebenenfalls substituierten Kohlenwasserstoffgruppen mit 1 bis 36 Kohlenstoffatomen ausgewählt sind, stehen,
- wobei R² und R³ zusammen einen das Stickstoffatom, an das sie gebunden sind, enthaltenden Ring bilden können, wobei der Ring gegebenenfalls substituiert ist und/oder ein zusätzliches Heteroatom enthält, und
- wobei R² und R³ nicht gleichzeitig für Wasserstoffatome stehen,
**dadurch gekennzeichnet, dass**
- das Amin (III) in einem organischen Lösungsmittel oder in der Diesterverbindung (II) gelöst wird,
- wenn das Amin (III) in einem organischen Lösungsmittel gelöst wird, die Diesterverbindung (II) zu der das Amin (III) und die basische Verbindung enthaltenden Reaktionsmischung gegeben wird,
- wenn das Amin (III) in der Diesterverbindung (II) gelöst wird, die basische Verbindung zu der das Amin (III) und die Diesterverbindung (II) enthaltenden Reaktionsmischung gegeben wird,
- die Umsetzung bei der Temperatur zwischen 30°C und 130°C durchgeführt wird,
- das Amin (III) in einem molaren Überschuss im Bereich von 0,01 bis 50 %, bezogen auf die Diesterverbindung (II), vorliegt.

2. Verfahren nach Anspruch 1, bei dem A für eine verzweigte zweiwertige Alkylengruppe mit einer Zahl von Kohlenstoffatomen im Bereich von 2 bis 12, vorzugsweise im Bereich von 3 bis 6 Kohlenstoffatomen, steht.

3. Verfahren nach Anspruch 1 oder 2, bei dem die Gruppen R¹ gleich oder verschieden sind und für Kohlenwasserstoffgruppen mit 1 bis 16 Kohlenstoffatomen, die einen oder mehrere Substituenten tragen können, stehen.

4. Verfahren nach einem der Ansprüche 1 bis 3, bei dem die Gruppen R¹ gleich oder verschieden sind und aus Alkyl-, Alkenyl-, Cycloalkyl-, Aryl- und Arylalkylgruppen ausgewählt sind, wobei die Gruppen einen oder mehrere Substituenten tragen können.

5. Verfahren nach einem der Ansprüche 1 bis 4, bei dem R¹ aus Methyl-, Ethyl-, n-Propyl-, Isopropyl-, n-Butyl-, Isobutyl-, tert-Butyl-, sec-Butyl-, n-Pentyl-, Isopentyl-, sec-Pentyl-, Cyclopentyl-, n-Hexyl-, Isohexyl-, sec-Hexyl-, Cyclohexyl-, Methylcyclohexyl-, 2-Ethylbutyl-, 3-Methylpentyl-, n-Heptyl-, Isoheptyl-, sec-Heptyl-, 3-Methylhexyl-, 4-Methylhexyl-, 1-Ethylpentyl-, 2-Ethylpentyl-, 3-Ethylpentyl-, n-Octyl-, Isooctyl-, 3-Methylheptyl-, n-Nonyl-, n-Decyl-, Undecyl-, n-Dodecyl-, Tridecyl-, Tetradecyl- und Pentadecylgruppen ausgewählt ist.

6. Verfahren nach einem der Ansprüche 1 bis 5, wobei es sich bei der Diesterverbindung der Formel (II) um ein Gemisch von Diesterverbindungen der folgenden Formeln (II.1), (II.2) und (II.3) handelt :
R¹OOC-CH(CH₃)-CH₂-CH₂-COOR¹ (II.1)
R¹OOC-CH(CH₂-CH₃)-CH₂-COOR¹ (II.2)
R¹OOC-CH₂-CH₂-CH₂-CH₂-COOR¹ (II.3)
wobei R¹ wie in einem der Ansprüche 1 bis 5 definiert ist.

7. Verfahren nach Anspruch 6, bei dem das Gemisch von Diesterverbindungen der folgenden Formeln (II.1), (II.2) und (II.3) die folgende Zusammensetzung aufweist :
- 75 bis 95 Gew.- % der Verbindung der Formel (II.1), vorzugsweise 85 bis 95 Gew.- %,
- 3 bis 23 Gew.- % der Verbindung der Formel (II.2), vorzugsweise 4 bis 14 Gew.- %,
- 0,1 bis 10 Gew.- % der Verbindung der Formel (II.3), vorzugsweise 0,1 bis 3 Gew.- %.

8. Verfahren nach Anspruch 6 oder 7, bei dem die Gruppen R¹ für Methylgruppen stehen.

9. Verfahren nach Anspruch 1, bei dem die Gruppen R² und R³ gleich oder verschieden sind und aus Alkyl-, Alkenyl-, Cycloalkyl-, Aryl- und Arylalkylgruppen ausgewählt sind, wobei die Gruppen einen oder mehrere Substituenten tragen können.

10. Verfahren nach Anspruch 9, bei dem R² und R³ gleich oder verschieden sind und aus Methyl-, Ethyl-, Hydroxyethyl-, n-Propyl-, Isopropyl-, n-Butyl-, Isobutyl-, tert-Butyl-, n-Pentyl-, Isoamyl-, Hexyl- und Cyclohexylgruppen ausgewählt sind.

11. Verfahren nach Anspruch 1, bei dem R² und R³ zusammen einen das Stickstoffatom, an das sie gebunden sind, enthaltenden Ring mit 5 oder 6 Atomen bilden, wobei es sich bei einem der Atome des Rings um ein weiteres Heteroatom, wie beispielsweise Sauerstoff, handeln kann.

12. Verfahren nach Anspruch 11, bei dem R² und R³ zusammen einen Ring, der aus Morpholin, Piperidin und Piperazin ausgewählt ist, bilden.

13. Verfahren nach einem der Ansprüche 1 bis 12, bei dem das Amin der Formel (III) in einem organischen Lösungsmittel gelöst wird.

14. Verfahren nach Anspruch 13, bei dem das organische Lösungsmittel aus Alkoholen und Ethern, vorzugsweise aus Methanol, Ethanol, Tetrahydrofuran (THF) und Mischungen davon, ausgewählt wird.

15. Verfahren nach einem der Ansprüche 1 bis 14, bei dem es sich bei der basischen Verbindung um ein Alkali- oder Erdalkalimetallalkoxid, das vorzugsweise aus Natriummethylat, Natrium- oder Kaliumethylat, Kalium-tert-butylat ausgewählt wird, Kalium- oder Natriumcarbonat, Alkyltitanate und Mischungen davon handelt.

16. Verfahren nach einem der Ansprüche 1 bis 15, bei dem die basische Verbindung in einer molaren Konzentration, bezogen auf den Diester, zwischen 0,01 und 20 %, vorzugsweise zwischen 3 und 10 %, eingetragen wird.

17. Verfahren nach einem der Ansprüche 1 bis 16, bei dem die Umsetzung bei einer Temperatur zwischen 40°C und 90°C und noch weiter bevorzugt zwischen 45°C und 65°C durchgeführt wird.

## Claims

1. A process for preparing an esteramide compound of following formula (I) :
R¹OOC-A-CONR²R³ (I)
comprising a reaction step between :
a diester compound of following formula (II) :
R¹OOC-A-COOR¹ (II)
and an amine of following formula (III) :
HNR²R³ (III)
in the presence of a basic compound,
formulae in which :
- A is a covalent bond or a linear or branched divalent alkylene group comprising a number of carbon atoms ranging from 1 to 12,
- R¹ is an optionally substituted hydrocarbon group, comprising from 1 to 36 carbon atoms,
- R² and R³, the same or different, are groups chosen from the hydrogen atom and the hydrocarbon groups, optionally substituted, comprising from 1 to 36 carbon atoms,
- R² and R³ being able together to form a ring comprising the nitrogen atom with which they are linked, the said ring possibly being substituted and/or comprising an additional heteroatom, and
- R² and R³ not simultaneously being hydrogen atoms,
**characterized by** the fact that :
- the amine (III) is dissolved in an organic solvent or in the diester compound (II),
- when the amine (III) is dissolved in an organic solvent, the diester compound (II) is added to the reaction mixture comprising the amine (III) and the basic compound,
- when the amine (III) is dissolved in the diester compound (II), the basic compound is added to the reaction mixture comprising the amine (III) and the diester compound (II),
- the reaction is conducted at a temperature of between 30°C and 130°C,
- the amine (III) is present in a molar excess relative to the diester compound (II) ranging from 0.01 to 50 %.

2. The process according to claim 1, wherein A is a branched divalent alkylene group comprising a number of carbon atoms ranging from 2 to 12, preferably ranging from 3 to 6 carbon atoms.

3. The process according to claim 1 or 2, wherein the R¹ groups, the same or different, are hydrocarbon groups comprising from 1 to 16 carbon atoms possibly carrying one or more substituents.

4. The process according to one of claims 1 to 3, wherein the R¹ groups, the same or different, are chosen from the groups consisting of the alkyl, alkenyl, cycloalkyl, aryl and arylalkyl groups, the said groups possibly carrying one or more substituents.

5. The process according to any of claims 1 to 4, wherein R¹ is chosen from the groups consisting of the methyl, ethyl, n-propyl, isopropyl, n-butyl, isobutyl, tert-butyl, sec-butyl, n-pentyl, isopentyl, sec-pentyl, cyclopentyl, n-hexyl, isohexyl, sec-hexyl, cyclohexyl, methylcyclohexyl, 2-ethylbutyl, 3-methylpentyl, n-heptyl, isoheptyl, sec-heptyl, 3-methylhexyl, 4-methylhexyl, 1-ethylpentyl, 2-ethylpentyl, 3-ethylpentyl, n-octyl, isooctyl, 3-methylheptyl, n-nonyl, n-decyl, undecyl, n-dodecyl, tridecyl, tetradecyl and pentadecyl groups.

6. The process according to any of claims 1 to 5, wherein the diester compound of formula (II) is a mixture of diester compounds of following formulae (II.1), (II.2) and (II.3) :
R¹OOC-CH(CH₃)-CH₂-CH₂-COOR¹ (II.1)
R¹OOC-CH(CH₂-CH₃)-CH₂-COOR¹ (II.2)
R¹OOC-CH₂-CH₂-CH₂-CH₂-COOR¹ (II.3)
where R¹ is such as defined in any of claims 1 to 5.

7. The process according to claim 6, wherein the mixture of diester compounds of formula (II.1), (II.2) and (II.3) has the following composition :
- from 75 to 95 % by weight of formula (II.1) compound, preferably 85 to 95 % by weight,
- from 3 to 23 % by weight of formula (II.2) compound, preferably 4 to 14 % by weight,
- from 0.1 to 10 % by weight of formula (II.3) compound, preferably 0.1 to 3 % by weight.

8. The process according to claim 6 or 7, wherein the R¹ groups are methyl groups.

9. The process according to claim 1, wherein the groups R² and R³, the same or different, are chosen from the groups consisting of the alkyl, alkenyl, cycloalkyl, aryl and arylalkyl groups, the said groups possibly carrying one or more substituents.

10. The process according to claim 9, wherein R² and R³, the same or different, are chosen from the groups consisting of the methyl, ethyl, hydroxyethyl, n-propyl, isopropyl, n-butyl, isobutyl, tert-butyl, n-pentyl, isoamyl, hexyl and cyclohexyl groups.

11. The process according to claim 1, wherein R² and R³ together form a ring with 5 to 6 atoms, comprising the nitrogen atom with which they are linked, one of the atoms of the ring possibly being another heteroatom, such as oxygen for example.

12. The process according to claim 11, wherein R² and R³ together form a ring chosen from a morpholine, a piperidine and a piperazine.

13. The process according to any one of claims 1 to 12, wherein the amine of formula (III) is in solution in an organic solvent.

14. The process according to claim 13, wherein the organic solvent is chosen from alcohols and ethers, preferably from methanol, ethanol, tetrahydrofuran (THF) and mixtures thereof.

15. The process according to any one of claims 1 to 14, wherein the basic compound is an alkoxide of an alkaline metal or alkaline-earth metal, preferably chosen from among sodium methylate, sodium or potassium ethylate, potassium terbutylate; potassium or sodium carbonate, alkyl titanates and mixtures thereof.

16. The process according to any one of claims 1 to 15, wherein the basic compound is added at a molar concentration relative to the diester of between 0.01 and 20 %, preferably between 3 and 10 %.

17. The process according to any one of claims 1 to 16, wherein the reaction is conducted at a temperature of between 40°C and 90°C and further preferably between 45°C and 65°C.
